(19) **Europäisches Patentamt / European Patent Office / Office européen des brevets**

(11) **EP 1 161 688 B1**

(12) **EUROPEAN PATENT SPECIFICATION**

(45) Date of publication and mention
of the grant of the patent:
**28.02.2007 Bulletin 2007/09**

(51) Int Cl.:
***G01N 33/92*** *(2006.01)*     ***G01N 33/543*** *(2006.01)*

(21) Application number: **00909610.8**

(22) Date of filing: **14.03.2000**

(86) International application number:
**PCT/IL2000/000158**

(87) International publication number:
**WO 2000/055623 (21.09.2000 Gazette 2000/38)**

(54) **COLORIMETRIC DETECTION METHOD**

KOLORIMETRISCHES BESTIMMUNGSVERFAHREN

PROCEDE DE DETECTION COLORIMETRIQUE

(84) Designated Contracting States:
**DE FR GB IT SE**

(30) Priority: **15.03.1999 IL 12900399**

(43) Date of publication of application:
**12.12.2001 Bulletin 2001/50**

(73) Proprietor: **BEN-GURION UNIVERSITY OF THE NEGEV**
**84105 Beer-Sheva (IL)**

(72) Inventor: **JELINEK, Raz**
**71908 Reut (IL)**

(74) Representative: **Jump, Timothy John Simon et al**
**Venner Shipley LLP**
**20 Little Britain**
**London EC1A 7DH (GB)**

(56) References cited:
**WO-A-97/27316**     **WO-A-98/39632**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European Patent Convention).

**Description**

**Field of the Invention**

The present invention relates to a method for the detection of species in a sample. More specifically, the present invention provides a. method for detecting an analyte in a liquid sample by a color transition occurring in the sample.

**Background of the Invention**

Various methods are known in the art for the detection of chemical and biological species in a sample. The methods that are frequently used in industrial or medical applications are based on conventional electrochemical and spectroscopic means. Recently, the art has been acknowledging the potential of polymers characterized by an absorption band which may be shifted from a first wavelength in the visible region to a second wavelength in a visible region, as possible detectors of chemical species in a sample. The color change that said polymers exhibit by virtue of their backbone being structurally perturbed in the presence of the analyte of interest, allows the identification of said analyte in the sample.
Attempts have been made to expand the utility of said polymers by providing particles composed therefrom, together with materials that can be chemically modified in the presence of the analyte. The chemical modification of said materials causes the perturbation in the polymer backbone, resulting in a color change, which indicates the presence of the analyte. WO 98/39632 and WO 97/27316 disclose a method for detecting the presence of an analyte based on a biopolymeric material containing a polymer having the properties described above, associated with a lipid. The color change is induced by the analyte interacting with a ligand that is covalently linked to the lipid in WO 97/27316, and by the analyte chemically reacting with the lipid, for example, by an enzyme causing a lipid cleavage, in WO 98/39632.
It is an object of the present invention to provide a method for detecting analytes that cannot react chemically with said polymers or lipids.

**Summary of the Invention**

It has been surprisingly found by the inventors that an aggregate particle comprising lipids and a polymer, said polymer having an absorption band which may be shifted from a first wavelength in the visible region to a second wavelength in the visible region, can be used to detect analytes without a chemical reaction occurring between the analyte and said lipids or said polymer. When brought into contact with a sample containing the analyte, said aggregate particle exhibits a color transition signaling the presence of the analyte, albeit the fact that the analyte does not react chemically with the components of the polymeric matrix. Hereinafter, this aggregate particle will be referred to as a polymeric matrix comprising lipids.
In one aspect, the present invention provides a method for detecting the presence of an analyte in a sample, said analyte being chemically non-reactive with lipids or with a polymer having an absorption band which may be shifted from a first wavelength in the visible region to a second wavelength in the visible region; which comprises:

   a) providing a polymeric matrix comprising said lipids and said polymer;
   b) introducing into said sample or into said polymeric matrix means enabling said analyte to cause a non-chemical change in said polymeric matrix; and
   c) contacting the sample with the polymeric matrix and observing a color transition of the matrix, indicating the presence of the analyte.

By the term "said analyte being chemically non-reactive with lipids or with a polymer" is meant an analyte which does not participate in a chemical reaction with either lipids or the polymer having the optical properties defined above. By the term "chemical reaction" is meant a reaction involving chemical cleavage, e.g., lipids cleavage, or any mode of specific interaction, or molecular recognition, between the lipids, or specific portions of the lipids, and the analyte, such as, for example, specific interaction between the analyte and the head groups of the lipids. Accordingly, the term "non-chemical change in the polymeric matrix" defines a change that is not associated with a chemical reaction as defined above.
The means allowing the analyte to cause a non-chemical change in the polymeric matrix are either ionophores or a receptor linked to a spacer arm located within the lipid domain of the polymeric matrix.
It has been found by the inventors that the polymeric matrix according to the present invention is capable of detecting ions in the tested sample, particularly metal cations, in the presence of ionophores.
It has also been found by the inventors that the presence of a receptor alone, in association with the polymeric matrix, is generally not sufficient to induce (by virtue of specific binding of an appropriate biological ligand to said receptor) the color transition of the polymeric matrix. According to the present invention, the means allowing a biological ligand to cause non-chemical changes in the lipids (for example, structural rearrangements), whereby a color transition is exhibited by the polymeric matrix, are provided by a receptor having the capability to bind said ligand, said receptor being linked to a spacer arm located within the lipid domain of said matrix. The spacer arm is preferably a peptide that is at least partially hydrophobic, or one or more alkyl chains. In the latter case, the receptor is linked to said one or more alkyl chains synthetically. Thus, by the term "receptor" is here meant a molecular structure having the ability to specifically bind a ligand of interest, wherein, according to the present invention, said receptor is bound to the matrix through said spacer arm. The receptor may include one or more epitopes, antigens and antibodies.

Polymers having an absorption band that may be shifted from a first wavelength in the visible region to a second wavelength in a visible region, which are suitable for forming the polymeric matrix according to the present invention, contain π-conjugated electronic systems, namely, these polymers contain conjugated double bonds and triple carbon-carbon bonds. The most preferred polymer according to the present invention is polydiacetylene, obtained by the polymerization of monomers selected from the group of diacetylene lipid acids and diacetylene derivatives, such as, for example, tricosadiynoic acid, tricosadiynoic methyl esters, pentacosadiynoic acid and pentacosadiynoic methyl esters. In the absence of the analyte, polydiacetylene absorbs light at a first wavelength which is approximately 640 nm. In the presence of the analyte, the absorption is shifted to a second wavelength, which is approximately 520 nm. Thus, a color transition from blue to red indicates the presence of the analyte. The lipids according to the present invention are preferably selected from the group consisting of phospholipids, particularly phosphoglycerides, sphingolipids, ceramides and glycolipids. Compositionally, the preferred molar ratio between the lipid and the polymer in the matrix is higher than 1:5. Typically, an intense color transition in the matrix is observed for a molar ratio which is about 2:3. Higher ratios may diminish the intensity of the color transition.

According to a first embodiment of the present invention, the analyte is an ion, more preferably a metal cation, and the means allowing said ion to cause a non-chemical change in the lipids are ionophores. Ionophores are molecules that selectively bind cations and transport them across a membrane. Quantitatively, the molar ratio between the ionophores and the lipids is between 1:3 to 1:1, more preferably 1:2.

According to a second embodiment of the present invention, the analyte to be detected is a peptide. According to one variant of the invention, the peptide is a short membrane peptide containing no more than 50 amino acids. In this variant, no means such as those required in step b) of the method are necessary for the detection of said short peptide. In another variant, the peptide is a membrane protein.

Surprisingly, it has been found that the detection is sensitive to the substitution, omission or addition of one or more amino acids in the peptide. In this context, the peptide will be referred to as the "native peptide", whereas the term "analogue" will indicate a peptide in which one or more amino acid in the native amino acid sequence was substituted, omitted or added. Thus, distinguishing between a native peptide and an analogue thereof comprises:

a) providing a polymeric matrix comprising lipids and a polymer, said polymer having an absorption band which may be shifted from a first wavelength in the visible region to a second wavelength in a visible region;

b) contacting the polymeric matrix with a sample suspected of containing said peptide or an analogue thereof, and comparing the color observed with the color expected in the presence of the native peptide, to determine whether the native peptide or an analogue thereof is present in the sample.

Distinguishing between a first analogue of a native peptide and a second analogue thereof comprises the steps of:

a) providing a polymeric matrix comprising lipids and a polymer, said polymer having an absorption band which may be shifted from a first wavelength in the visible region to a second wavelength in a visible region;

b) contacting the polymeric matrix with a sample suspected of containing said first analogue or said second analogue, and comparing the color observed with the color expected in the presence of said first analogue or said second analogue, to determine which analogue is present in the sample.

The surprising sensitivity to minor alterations in the peptide sequence provides a tool for evaluating the biological activity of various analogues of a given peptide based on the degree of color transitions exhibited by the polymeric matrix in their presence, which comprises the steps of:

a) providing a polymeric matrix comprising lipids and a polymer, said polymer having an absorption band which may be shifted from a first wavelength in the visible region to a second wavelength in a visible region;

b) contacting the polymeric matrix with a sample containing said analogue, and comparing the color observed with the color expected in the presence of the native peptide;

c) assessing the difference between the color observed and the color expected, to evaluate the similarity between the biological activity of said analogue and said peptide in membrane-related systems.

According to another embodiment of the present invention, the analyte is a biological ligand that is preferably selected from the group consisting of antibodies, antigens and epitopes. The detection of these biological ligands is carried out by introducing into the polymeric matrix a receptor having the capability to bind the ligand, wherein said receptor is linked to a spacer arm that is preferably a peptide which is at least partially hydrophobic, or one or more alkyl chains, the latter being synthetically linked to said receptor.

## Brief Description of the Drawings

Figure 1 is a schematic representation of the polymeric matrix according to the present invention (PC indicates a specific headgroup, namely, phosphtydilcholine headgroup).

Figure 2 is a graph showing the color response of the polymeric matrix according to the present invention in the presence of metal cations and the ionophore valinomycin.

Figure 3 is a graph showing the color response of the polymeric matrix according to the present invention in the presence of metal cations and the ionophore monensin.

Figure 4 is a graph showing the color response of the polymeric matrix according to the present invention in the presence of metal cations and the ionophore A23187.

Figure 5 depicts the selectivity of the polymeric matrix and a ionophore in a $K^+$ / $Na^+$ mixture.

Figure 6 shows the UV/vis absorption spectra of a solution containing the polymeric matrices before and after the addition of the peptide melittin.

Figure 7 shows the colorimetric response of several solutions containing polymeric matrices comprising different lipids as a function of the concentration of the peptide melittin added to the solution.

Figure 8 shows a comparison between dimyristoylphosphocholine (DMPC)-containing polymeric matrix and dipalmitoylphosphcholine (DPPC)- containing polymeric matrix in the detection of melittin at temperature of 27°C.

Figure 9 illustrates the detection of an analogue of melittin (melittin-L9W) according to the present invention.

Figure 10 illustrates the detection of analogues of magainin (magainin-K11E and magainin-K10E,K11E) according to the present invention.

Figure 11 illustrates the detection of an analyte which is an antibody.

Figure 12 shows the C-13 NMR spectra of polymeric matrix according to the present invention, in the presence the ionophore valinomycin and in the presence of $K^+$.

## Detailed Description of Preferred Embodiments

The polymeric matrix according to the present invention is preferably prepared by dissolving the lipids and a monomer capable of forming the polymer having the optical properties described above in an organic solution, such as chloroform:ethanol mixture (1:1 v/v), in a molar ratio which is in the range between 1:5 to 2.2:3, a ratio of about 2:3 being most preferred. Following filtration of the lipids and the monomer, and removal of residues of the organic solvent therefrom, water is added to yield an aqueous solution containing the lipids and the monomer in a concentration which is preferably between 0.5 mM to 5mM, more preferably between 1 to 2 mM. Sonication of the aqueous mixture is carried out at a temperature in the range between 60 to 85°C, preferably at about 70°C. The duration of the sonication is dependent on the power generated by the sonicator. Typically, the sonication is carried out for 2 to 10 minutes. For example, for a sonicator of 100W power, with short intermission between the sonication pulses, the sonication lasted 5 minutes. The mixture is then allowed to cool down at temperature of about 4°C for at least 12 hours. Subsequently, the polymerization of the monomer is initiated by means of irradiation at an appropriate wavelength (254 nm for about 10 to 60 seconds, in the case of polydiacetylene), resulting in the formation of a polymeric matrix containing the lipids.

The preferred polymer according to the present invention is polydiacetylene obtained by polymerization of the monomer 10,12-tricosadiyoic acid. The preferred lipids according to the present invention are selected from the group consisting of dimirystoylphosphatydilcholine (DMPC), dipalamitoylphophatidylchline (DPPC), dimirystoylphosphoethanolamine (DMPE), natural phosphatydilcholine (PC), dimirystoylphosphatidylcholine (DMPG), cardiolipin, dimyristoylphophatyldiserine (DMPS), sphingomyelin, ceramide, galactosylderamide, lipopolysaccharide (LPS) and cholesterol, or mixtures thereof.

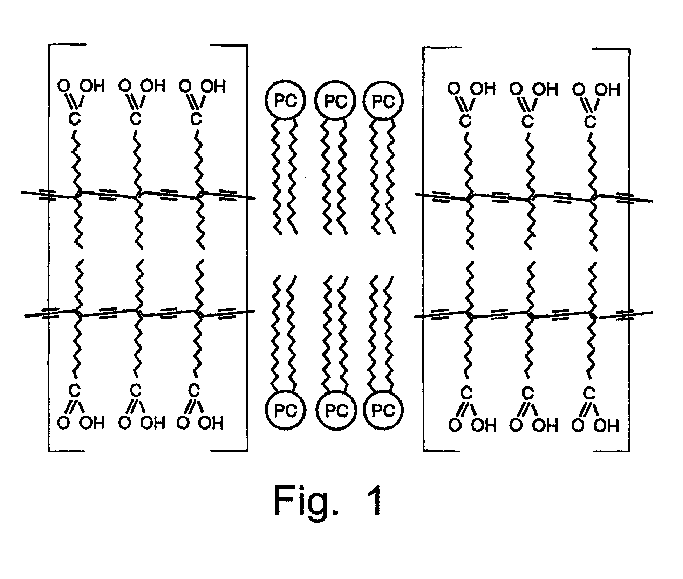

Figure 1 provides a schematic representation of the polymeric matrix according to the present invention, showing the lipid chains embedded within the conjugated polymer. The morphologies of the polymeric matrix are generally ellipsoidal and spherical, and may include tubular shapes, rectangular sheets, as well as elongated and irregular shapes. Typically, the size distribution of the polymeric matrices is between 100 nm and 1000 nm length.

The method of detection according to the present invention is preferably carried out by contacting a solution that contains the polymeric matrix and the means allowing the analyte to cause a non-chemical change in the polymeric matrix, with a sample suspected of containing the analyte, at a temperature in the range between 25 to 30°C, more preferably at 27°C. The solution containing said polymeric matrix and said means is generally mixed with an equal volume of a buffer at pH of 8.0, before the introduction of said sample thereto.

According to a preferred embodiment of the present invention, the analyte is a metal cation. To carry out the detection of a metal cation, a ionophore is added to a solution comprising the polymeric matrix, before or after said solution is contacted with the sample that is suspected to contain the analyte. Quantitatively, the molar ratio between the ionophore and the phospholipids comprised in the matrix is between 1:4 and 1:1, more preferably about 1:2. The ionophore is preferably selected from the group consisting of valinomycin, monensin and the divalent pyrrole A23187. The utilization of a particular ionophore for the purpose of detection is based on its selective affinities towards the cations suspected of being present in the sample. Preferably, metal cations to be detected by the present invention are selected from the group consisting of cations of alkaline metals, cations of alkaline earth metals and cations of transition metals.

It has been found that the presence of several cations in the tested sample does not diminish the sensitivity of the detection method according to the present invention, towards the analyte of interest. This finding is quite surprising, since it is known in the art that the presence of several cations tends to mask the cation of interest, thereby reducing the sensitivity of the detection.

The analyte to be detected can be a peptide that contains no more than 50 amino acids.

Quantitatively, the molar ratio between the peptide and phospholipids is in the range between 1:1000 to 1:10, preferably about 1:100.

The detection method is sensitive to the amino acid sequence of the peptides. The sensitivity of the method is reflected by either an analogue causing a different blue-to-red color transition in the polymeric matrix, compared with the native peptide, or by the analogue failing to induce any color transition, the polymeric matrix maintaining its blue color.

The method can distinguish between melittin and analogues thereof, for example, an analogue in which lysine-7 has been substituted with leucine, an analogue in which leudne-9 has been replaced with tryptophan and an analogue wherein tryptophan-19 has been omitted. In these cases, the polymeric matrix appears more reddish in the presence of the analogues compared with the native peptide.

The method can distinguish between magainin II and analogues thereof, for example, an analogue in which lysine-11 has been substituted with glutamic acid, and an analogue in which both lysine-10 and lysine-11 have been similarly replaced with glutamic acid. When brought into contact with said analogues, the polymeric matrix maintained its blue color and did not exhibit the blue-to-red color transition induced by native magainin II. The present invention also allows to distinguish between magainin II and an analogue thereof in which phenylalanine-12 has been substituted with tryptophan. It has been found that said analogue does induce a color change, albeit more moderate than native magainin II.

The method distinguishes between alamethicin and analogues thereof, for example, an analogue in which proline has been moved from position 14 to position 12 in the sequence. When brought into contact with said analogue, the polymeric matrix maintained its blue color and did not exhibit the blue-to-red color transition induced by native magainin II.

The color changes induced in the solutions containing the polymeric matrix occur within seconds after addition of the peptides. The speed of the assay, combined with the simplicity of detection, would be an important advantage over conventional anti-bacterial assays, which require, in general, much longer times to carry out The assay is robust and can be easily expanded to include a variety of membrane models; this colorimetric assay can be applied for rapid screening of anti-bacterial peptide activities, and could provide structural and functional information on peptide-membrane interactions and mechanisms of membrane permeability. Because of the direct relationships between the peptide sequences and color change of the polymeric matrix, the method may be used in biopharmaceutical applications and biochemical research and development Among the potential applications are: high-throughout screening of antibiotic peptides libraries, diagnostic kits for antibiotic peptides, development of anti-microbial drugs, which induce membrane perturbations and/or lysis, and elucidating the factors affecting peptide-membrane interactions.

According to another embodiment of the present invention, the analyte is a biological ligand that is preferably selected from the group consisting of antibodies, antigens and epitopes. The detection of these biological ligands is carried out by adding to the polymeric matrix a receptor having the capability to bind the ligand, said receptor being linked to a spacer arm located within the lipid domain of said matrix. According to a preferred variant, said spacer arm is a synthetic membrane peptide to which a receptor is synthetically attached, said peptide being at least partially hydrophobic, containing about 8 to 70 amino acids. According to another preferred variant, said spacer arm is a naturally occurring membrane protein a portion of which acts as a receptor. According to another preferred variant, said spacer arm is a recombinant membrane protein displaying the peptide receptor. According to another preferred variant, the spacer arm is one or more alkyl chains, preferably $C_{14}$-$C_{21}$ alkyl chains, which are synthetically linked to the receptor.

Methods for attaching a receptor to a peptide, including a protein, or to an alkyl chain are known in the art. One such method, for example, is known as the phage-display peptide epitope library (Smith, G.P., Scott, J.K. (1993) "Libraries of peptides and proteins displayed on filamentous phage", Methods Enzymol. vol 217, 228-257. Other methods concerned with the attachment of receptors to alkyl chains are described in Toledano, O., Magdassi, S. (1997) "Formation of surface active gelatin by covalent attachement of hydrophobic chains", J. Colloidal Interf. Sci. vol. 193, 172-175.

The spacer arm and the receptor attached thereto are added to the organic solution containing the lipids and the monomer, the molar ratio spacer arm:lipid being in the range between 1:50 to 1:500, preferably about 1:200. The organic solution is subsequently treated in the manner described above, to obtain an aqueous solution containing the polymeric matrix to which the receptor is bound through said spacer arm.

Preferably, the biological ligand is an antibody and the spacer arm is a synthetic membrane peptide to which a receptor is synthetically attached, or a naturally occurring membrane protein a portion of which acts as a receptor. Specific epitope-antibody and/or antigen-antibody interactions have been detected in the following generic systems: antibodies raised against fd filamentous bacteriophage, and antibodies directed against the short "FLAG" peptide with the sequence DYKDDDDK, attached to the N-terminus of a transmembrane peptide domain. In the first system, the p8 helical membrane protein (which consists of more than 95% of the bacteriophage mass), was introduced into an organic solution containing the phospholipids and the monomer, as explained before. Following the procedure of sonication and polymerization, a blue-to-red color change has been detected only upon addition of the anti-fd antibodies to aqueous solution containing the polymeric matrix. Similarly, soluble anti-FLAG antibodies have been shown to induce colorimetric transitions in solutions of polymeric matrix containing the FLAG peptide covalently bonded to the N-terminal of the transmembrane sequence.

The quantitative measure of the color transition exhibited by the polymeric matrix in the presence of the analyte is given by the Colorimetric Response (CR), defined as:

$$CR = (PB_0 - PB_I) / PB_0$$

Where

$$PB = A_{first\ wavelength} / (A_{first\ wavelength} + A_{second\ wavelength})$$

A is the absorbance measured at either the first wavelength in UV-vis spectrum or the second wavelength (640 nm and 500 nm for polymeric matrix based on polydiacetylene). $PB_0$ is the ratio calculated for the control sample (without the analyte), while $PB_I$ is the value calculated for the solution containing the polymeric matrix according to the present invention and the analyte. UV-vis measurements were carried out at 27°C on a Hewlett-Packard 8452A diode array spectrophotometer, using a 1cm optical path cell.

The method according to the present invention is not limited to the qualitative detection of an analyte that is chemically non-reactive with lipids or with a polymer. By using calibration curves of colorimetric response versus analyte concentration, the quantity of analyte present in the tested sample may be determined.

The above and other characteristics and advantages of the invention will be further understood from the following illustrative and non-limitative examples.

### Examples

Preparation 1

Preparation of a polymeric matrix composed of the polymer polydiacetylene and the lipid dimyristoylphosphocholine (DMPC)

Synthetic dimyristoylphosphocholine (Avanti Polar Lipids, Alabaster, AL) and 10,12-tricosadiynoic acid (GFS Chemicals, Powell, OH) were dissolved in 1:1 chloroform:ethanol mixture and filtered through 0.8 micron filter. The phospholipid and the monomer were dried under vacuum for 3-4 hours. 2 ml of water was added to give a final concentration 0.8 mM lipid and 1.2 mM 10,12-tricosadiynoic acid. The mixture was subjected to sonication at a temperature of around 70°C by a probe sonicator exhibiting a power of 100W, for 5 minutes, using 45 seconds sonication pulses interrupted by 15 seconds intermissions. Following sonication, the solution was cooled at 4°C overnight and irradiated at 254 nm in a uv irradiation oven (cross linker) for 10-20 seconds in order to induce polymerization of the polydiacetylene. The solution acquired an intense blue color following irradiation.

Under similar conditions, polymer matrices were prepared, comprising polydiacetylene and the following lipids:

dipalamitoylphophatidylchline (DPPC);
dimirystoylphosphoethanolamine (DMPE);
natural PC;
dimirystoylphosphatidylcholine (DMPG);
cardiolipin,
DMPS;
Sphingomyelin;
Ceramide;
Galactosylderamide;
LPS; and
cholesterol.

Preparation 2

Preparation of a polymeric matrix comprising the polymer polydiacetylene, the lipid dimyristoylphosphocholine (DMPC) and a biological receptor which is a peptide epitope

The FLAG epitope, (DYKDDDDK), was chemically synthesized as the N-terminus of a generic transmembrane peptide sequence, using Fmoc-based solid phase peptide synthesis (SPPS). The overall sequence of the peptide was DYKDDDDKGKKLALALALALALALKKA-Amide. The peptide, dissolved in water, was mixed with a solution of chloroform: ethanol (1:1 v/v), containing dimyristoylphosphocholine (DMPC) and the monomer 10,12-tricosadiynoic acid, the molar ratio between the peptide, the phosphlipid and the monomer being 1:200:300. The mixture was then dried in a rotor-evaporator for 8 hours. Following the drying, water was added to obtain a solution containing the phospholipid and the monomer in concentrations of 0.4 mM and 0.6 mM, respectively. The aqueous solution of was then sonicated for 5 minutes (using a 100W probe sonicator) at a temperature of 70°C. Following sonication, the clear solution was cooled for 12 hours at 4°C. The solution was then brought to room temperature, and irradiated for 20 seconds at 254 nm, using a uv-oven (cross linker) operating at 0.8 J/cm$^2$ intensity. The solution acquired an intense blue color following irradiation.

Example 1

Detection of alkaline metal ions by a polymeric matrix comprising polydiacetylene and dimyristoylphosphocholine in the presence of the ionophore valinomycin

Into 0.2 mL of a solution prepared according to Preparation 1, was added 30 μL of trifluoroethanol containing the ionophore valinomycin. The concentration of the ionophore in the solution was 0.6 mM. Following the addition of a tris solution (0.2 mL) at pH of 8.5, the final concentration of the ionophore was 0.3 mM.

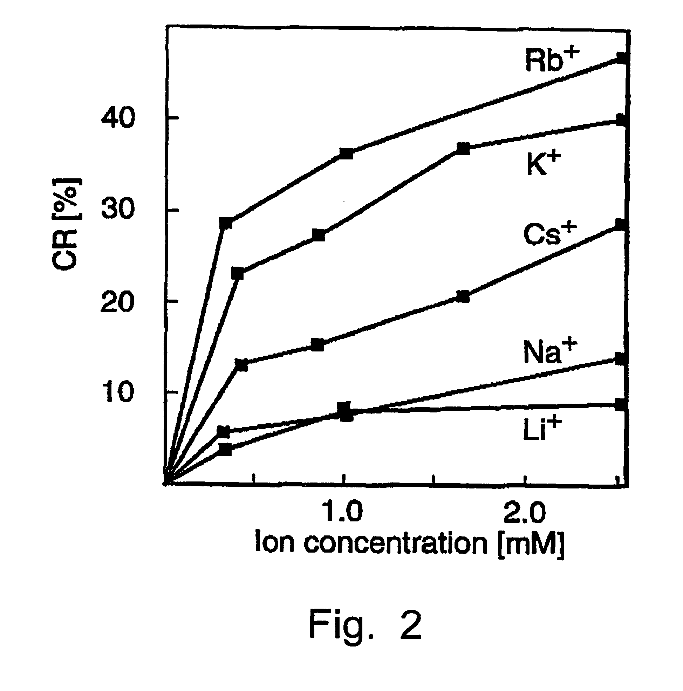

The solution was used for the detection of cations Rb+, K+, Cs+, Na+ and L+, and each of the samples tested contained a single cation in concentration of between 1 to 10 mM. The results are given in Figure 2, showing the colorimetric response versus the ion concentration. It is apparent from the graph that for the ionophore valinomycin, the solution exhibits the most pronounced red color in the presence of rubidium ions, followed closely by potassium. Furthermore, a more moderate colorimetric transition is induced in the solution after addition of Cs$^+$ ions, while Li$^+$ and Na$^+$

ions do not seem to initiate a blue-red color change at all.

Example 2

Detection of alkaline metal ions by a polymeric matrix comprising polydiacetylene and dimyristoylphosphocholine in the presence of the ionophore monensin

Into 0.2 mL of a solution prepared according to Preparation 1, was added 30 $\mu$L of trifluoroethanol containing the ionophore monensin. The concentration of the ionophore in the solution was 0.6 mM. Following the addition of a tris solution (0.2 mL) at pH of 8.5, the final concentration of the ionophore was 0.3 mM.

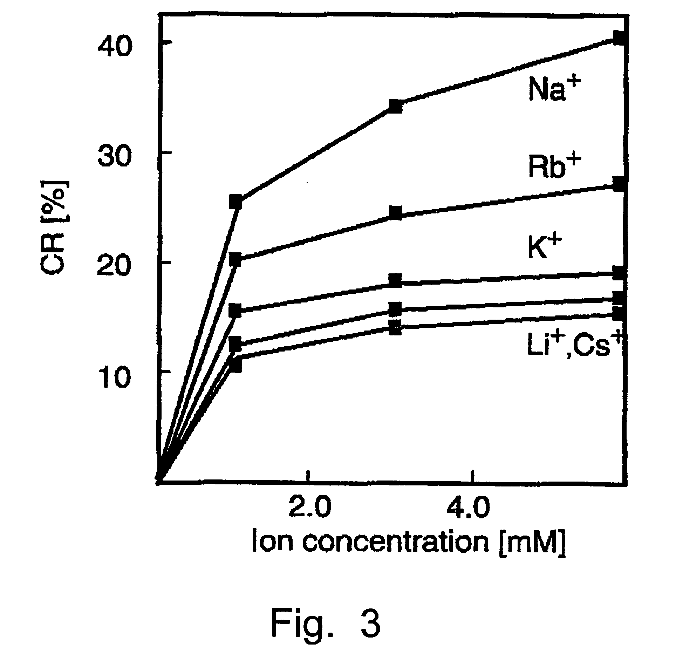

The solution was used for the detection of cations Rb+, K+, Cs+, Na+ and L+, and each of the samples tested contained a single cation in concentration of between 2 to 20 mM. The results are given in Figure 3, showing the colorimetric response versus the volume of the cation sample added to the polymeric matrix solution.

The greatest color change is observed in the presence of Na$^+$ ions. The color changes occur within seconds after addition of the ions to the polymeric matrix solution, and they reach stability within tens of seconds. The counter ions do not seem to affect the extent of color changes.

Example 3

Detection of different metal ions by a polymeric matrix comprising polydiacetylene and dimyristoylphosphocholine in the presence of the ionophore A23187

Into 0.2 mL of a solution prepared according to Preparation 1, was added 30 $\mu$L of trifluoroethanol containing the ionophore A23187. The concentration of the ionophore in the solution was 0.6 mM. Following the addition of a tris solution (0.2 mL) at pH of 8.5, the final concentration of the ionophore was 0.3 mM.

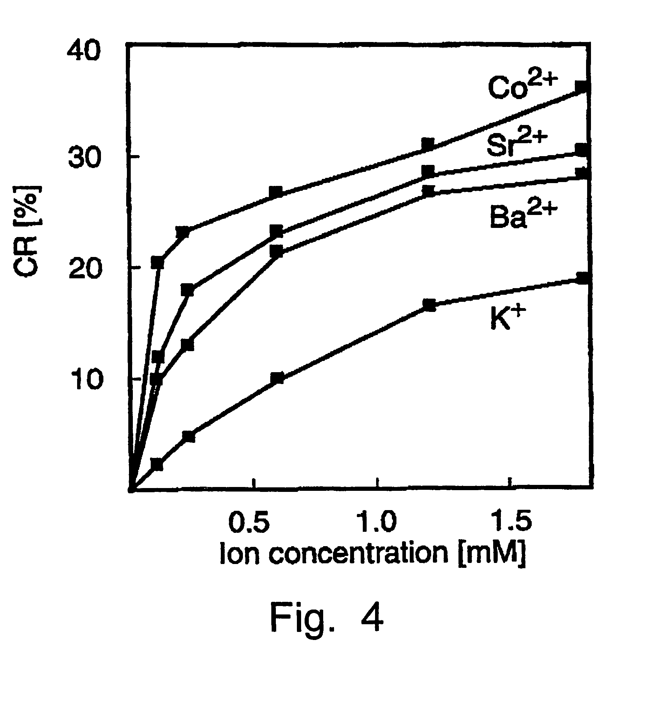

The solution was used for the detection of the cations Co$^{+2}$, Ba$^{+2}$, Sr$^{+2}$ and K+. Each of the samples tested contained a single cation in a concentration of between 0.5 to 5 mM . The results are given in Figure 4, showing the colorimetric response versus the concentration of the cation added to the polymeric matrix solution.

The results given in examples 1 to 3 illustrate another potential use of the polymeric matrix according to the present invention for screening ionophore-type molecules for their ionic binding.

Example 4

The detection of a specific metal cation by a polymeric matrix comprising polydiacetylene and dimyristoylphosphocholine and a inophores, in the presence of another metal cation

The detection method according to the present invention was tested with respect to a sample comprising a mixture of cations, K$^+$ and Na$^+$, in order to assess the selectivity of the method. The detection procedure was similar to the one described in the preceding examples.

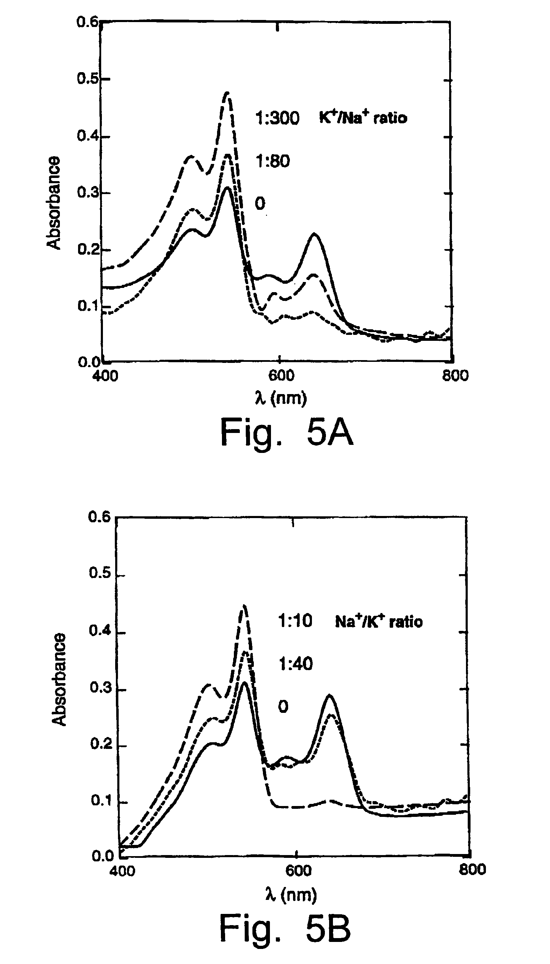

Figure 5 depicts the extent of K$^+$ /Na$^+$ selectivity of a solution containing polymeric matrix comprising polydiacetylene and dimyristoylphosphocholine and a ionophore. Figure 5(A) features the uv-vis absorption spectra of the polymeric matrix in the presence of ionophore valinomycin, when mixed with samples of sodium and potassium ions at different molar ratios. The uv-vis spectra indicate that, even at a Na$^+$ to K$^+$ molar ratio of 1:300, the polymeric matrix still successfully discriminate between potassium and sodium ions. The sensitivity towards the K$^+$ ions is apparent in the UV/vis spectra from the more intense "red band" at around 520 nm, compared to the "blue-band" at around 640 nm. The K$^+$/Na$^+$ selectivity observed in the mixed vesicles is significantly higher than values reported for various commercially available fluorescent ion-sensors. Similarly, Figure 5(B) demonstrates that the polymeric matrix together with the ionophore monensin exhibit selectivity of approximately 40 between Na$^+$ and K$^+$.

Example 5 (Illustrative)

Detection of the peptide melittin by a polymeric matrix comprising polydiacetylene and dimyristoylphosphocholine

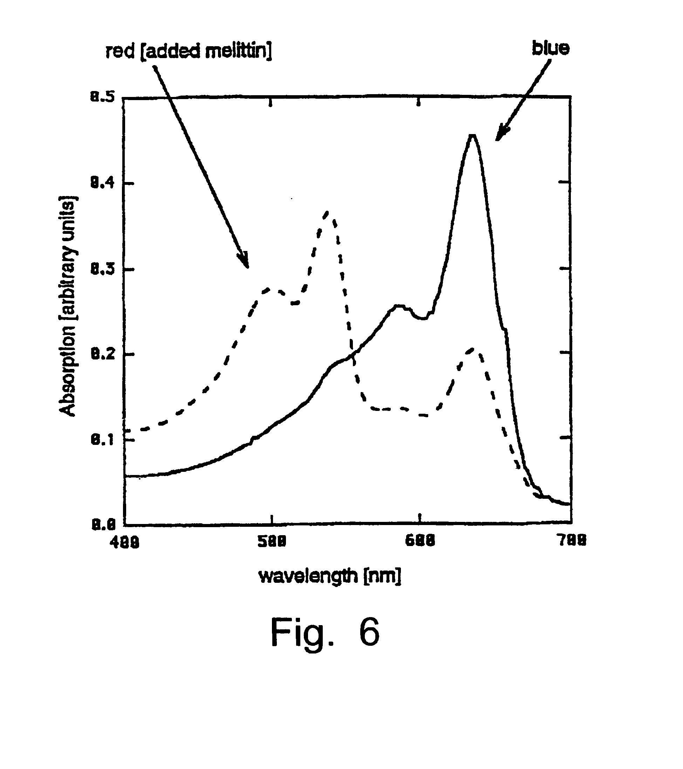

Into 0.2 mL of a solution prepared according to Preparation 1, comprising the polymeric matrix in a concentration of 1 mM was added 2 mM tris solution (0.2 mL). The pH of the solution was 8.5. Following the addition of the peptide melittin GIGAVLKVLTIGLPALISWIKRKRQQ (purified to >95% using reverse-phase HPLC) the solution was diluted to 1 cc, the final concentration of the peptide being 50 $\mu$M. Figure 6 shows the UV/vis absorption spectra of the solution containing the polymeric matrices before and after the addition of melittin, clearly illustrating the shift of the absorption band resulting in a blue-to-red color transition.

Example 6 (Illustrative)

Detection of the peptide melittin by a polymeric matrix comprising polydiacetylene and various phospholipids

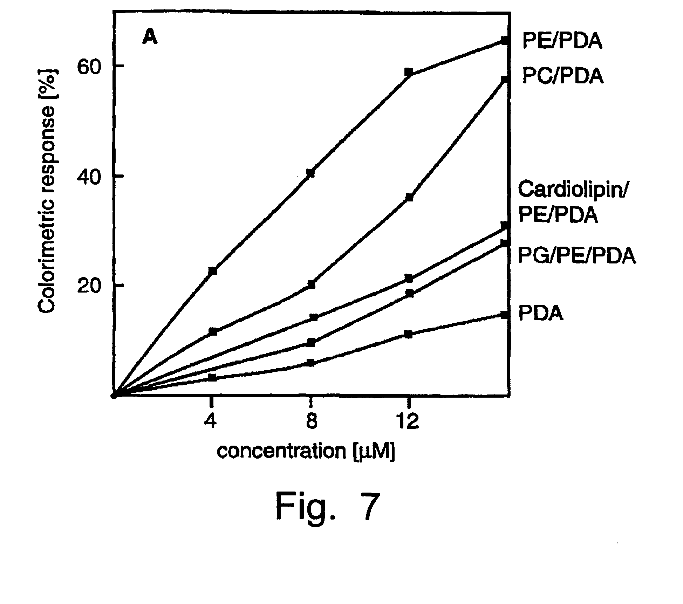

Solutions containing polymeric matrix based on polydiacetylene and various phospholipids were prepared according to the procedure of Preparation 1 and were tested for the detection of the peptide melittin, a representative anti-bacterial peptide, in the same manner described in Example 5. The results are shown in Figure 7, depicting the colorimetric responses of the polymeric matrix, measured at a temperature of 27 °C, as a function of the concentration of melittin. It is apparent from Figure 7 that the addition of melittin induces color changes not only in polymeric matrix containing dimyristoylphosphocholine (DMPC), but also in polymeric matrix incorporating dimiristoylphosphoethanolamine (DMPE), as well as mixtures of DMPE and dimiristoylphosphatidylglycerol (DMPG), or DMPE and cardiolipin. Low colorimetric response is detected upon addition of melittin to pure polidiacetylene (PDA) matrix (no lipids). This colorimetric response was treated as a "background" in the analysis of the results. The titration curves of melittin, shown in Figure 7, reveal that the polymeric matrix could detect melittin even in micro-molar concentrations (colorimetric response of less than 20%, for example, would look like a distinct color change even to a naked eye]. Such concentrations approach values employed in conventional anti-bacterial assays.

Example 7 (Illustrative)

Comparison between polymeric matrix comprising polydiacetylene and dimyristoylphosphocholine (DMPC) and polymeric matrix comprising polydiacetylene and dipalmitoylphosphcholine (DPPC) for the detection of melittin at a temperature of 27 °C

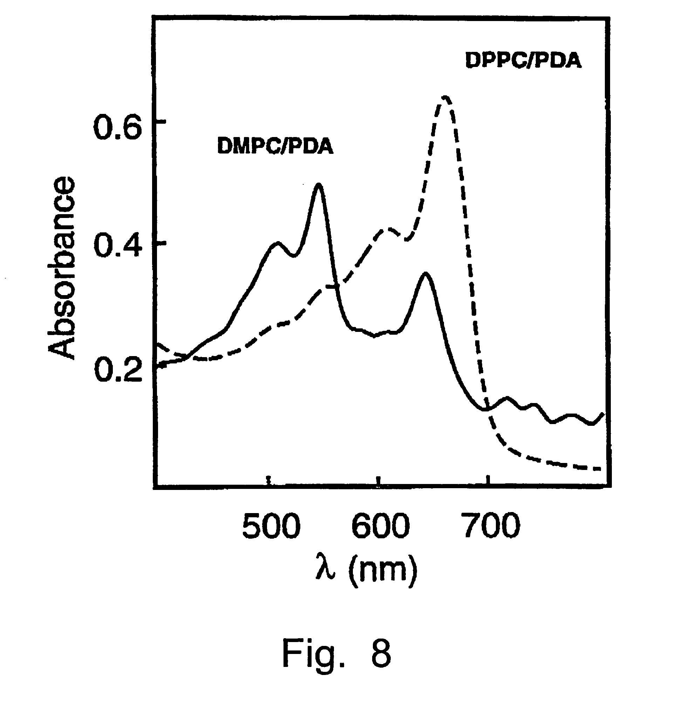

A solution containing a polymeric matrix based on polydiacetylene and dimyristoylphosphocholine (DMPC), and a solution containing a polymeric matrix based on polydiacetylene and dipalmitoylphosphcholine (DPPC), were prepared according to the procedure of Preparation 1, and were tested for the detection of the peptide melittin at a temperature of 27 °C, in the same manner described in Example 5. The results are shown in Figure 8, respectively. It is clearly apparent from the spectra that the detection of melittin is successfully accomplished using the polymeric matrix comprising polydiacetylene and dimyristoylphosphocholine (DMPC). However, the polymeric matrix comprising polydiacetylene and dipalmitoylphosphcholine (DPPC) fails to exhibit the blue-to-red color transition in the presence of melittin. The reason for this difference is that, contrary to DMPC, which forms a fluid lipid phase at 27 °C, DPPC is in the gel-phase at said temperature. Since melittin does not penetrate lipid bilayers in the gel phase, it cannot induce the structural rearrangement within the lipid, which is necessary to drive the perturbation of the polymer backbone. The comparison given in this example shows that the method according to the present invention is useful in detecting chemically non-reactive analytes by virtue of their ability to form physical interactions, rather than chemical interactions, within the lipids in the polymeric matrix.

Example 8 (Illustrative)

Detection of melittin analogues

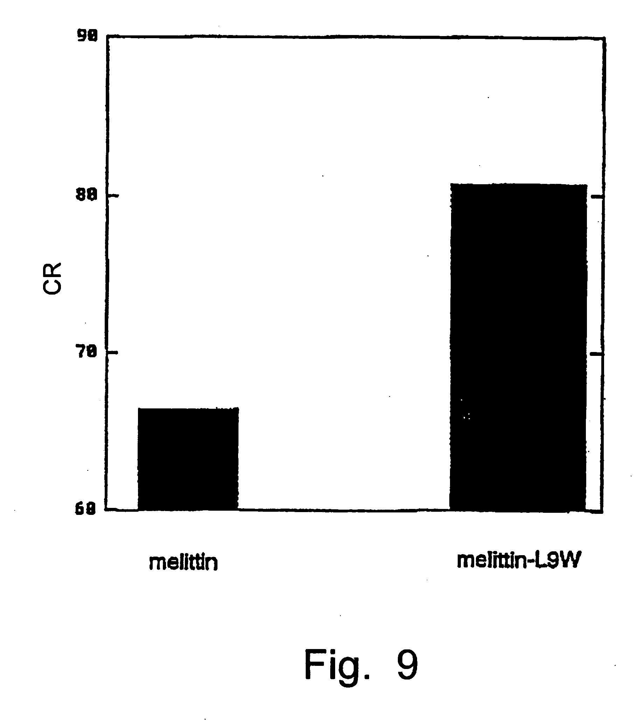

Into 0.2 cc of a solution prepared according to Preparation 1, comprising the polymeric matrix in a concentration of 1 mM, was added 2 mM tris solution (0.2 cc). The pH of the solution was 8.5. Following the addition of an analogue of the peptide melittin, in which Leu-9 was replaced with tryptophan (synthesized by Alpha Diagnostics Inc, San Antonio, TX, using standard Fmoc Chemistry and purified to >90% using reverse-phase HPLC), the solution was diluted to 1 cc. Figure 9 provides a comparison between the colorometric response of melittin and its analogue ($\lambda$=500 nm), clearly illustrating the ability of the present invention to distinguish between melittin and its analogue by virtue of the polymeric matrix appearing more reddish in the presence of said analogue.

Example 9 (Illustrative)

Detection of magainin II and analogues thereof

Into 0.2 cc of a solution prepared according to Preparation 1, comprising the polymeric matrix in a concentration of 1 mM, was added 2 mM tris solution (0.2 cc). The pH of the solution was 8.5. The solution was used for detecting the peptide magainin II (GIGKFLHSAKKFGKAFVGEIMNS, (purified to >95% using reverse-phase HPLC). Similar solutions were used to detect two analogues of magainin II:

A first analogue, in which lysine-11 was replaced with glutamic add;

A second analogue, in which both lysine-10 and lysine-11 were replaced with glutamic acid.

The analogues were synthesized by Alpha Diagnostics Inc, San Antonio, TX, using standard Fmoc Chemistry and purified to >90% using reverse-phase HPLC.

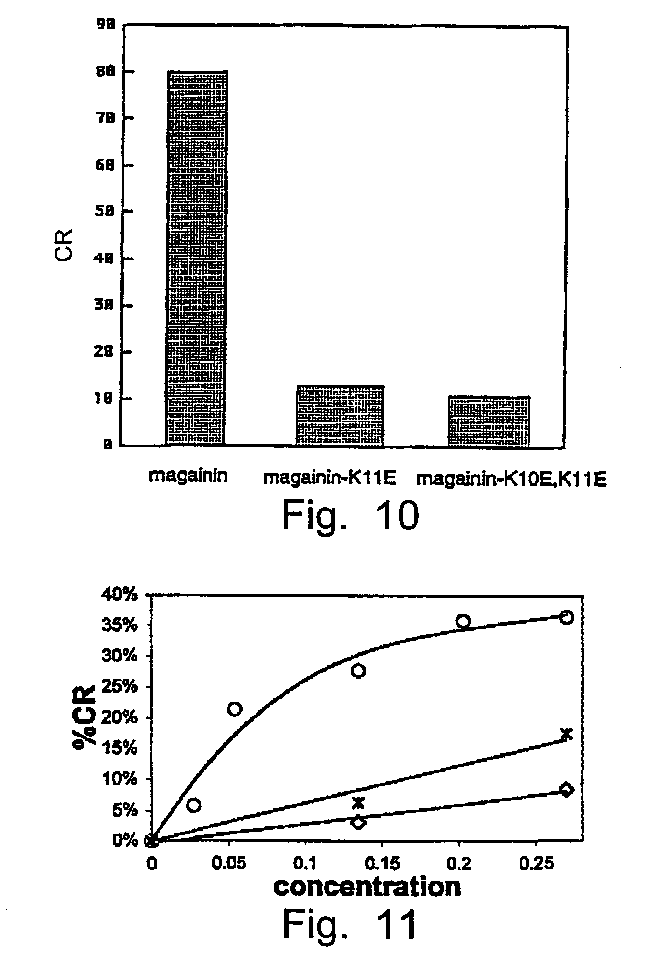

Following the addition of native peptide or the analogue, the solution was diluted to 1 cc. Figure 10 provides a comparison between the colorometric response of magainin and its analogues, clearly illustrating the ability of the present invention to distinguish between magainin and its analogues, by virtue of the polymeric matrix maintaining its blue color in the presence of said analogues.

Example 10

Detection of an antibody by a polymeric matrix comprising polydiacetylene, dimyristoylphosphocholine and the Flag epitope

Into a solution prepared according to Preparation 2 (0.2 mL) was added 0.2 mL of PBS, followed by the introduction of 20 μL of an antibody solution (4 mg/mL) dissolved in PBS buffer. The results are given quantitatively in Figure 10, showing the colorimetric response versus the concentration of the Flag antibody in the solution, denoted by circles. For the purpose of comparison, the colorimetric response was measured in two additional systems:

- Introduction of the FLAG antibody into a solution containing the polymeric matrix without the FLAG epitope (denoted by diamonds)
- Introduction of a different antibody (non-FLAG specific) into a solution containing the polymeric matrix with the FLAG epitope (denoted by crosses).

The comparison clearly illustrates the sensitivity of the detection method according to the present invention.

Example 11

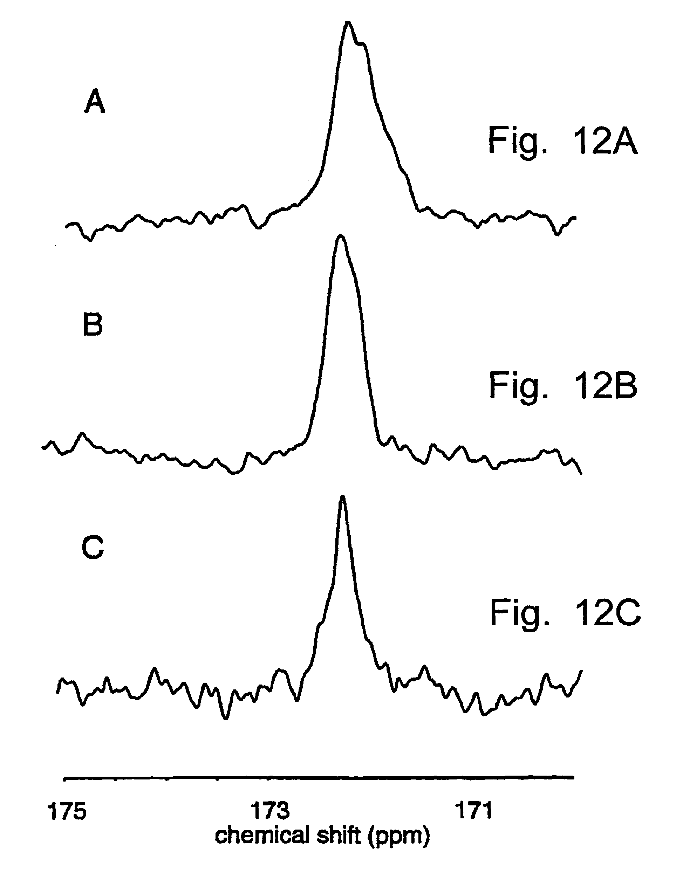

Figure 12 depicts C-13 NMR spectra acquired for solutions of polymeric matrix comprising DMPC and polydiacetylene, in which the DMPC molecules have been isotope-labeled with C-13 at the two carbonyl positions. The C-13 spectrum shown in Figure 12(A) corresponds to the two overlapping carbonyl signals from the DMPC molecules embedded within the polymeric matrix. Addition of valinomycin to the vesicles does not significantly affect the position or line-width of the C-13 signal, as shown in Figure 12(B). However, further addition of the analyte (KCl) to the polymeric matrix solution induces a noticeable narrowing of the C-13 resonance, Figure 12(C). The significant reduction of the line-width of the C-13 signal indicates a higher mobility of the carbonyls, which occurs in parallel with the blue-red color transition. It is clearly apparent that the introduction of the analyte does not shift the position of the C-13 signal, which would have been expected in the case of a chemical reaction between the lipids and the analyte.

**Claims**

1.  A method for detecting the presence of an analyte in a sample, said analyte being chemically non-reactive with lipids or with a polymer having an absorption band which may be shifted from a first wavelength in the visible region to a second wavelength in the visible region, which comprises:

    a) providing a polymeric matrix comprising said lipids and said polymer in an aqueous solution;
    b) introducing into said polymeric matrix said sample, and a means enabling said analyte to cause a change accompanied by a color transition in said polymeric matrix; wherein no covalent bond is formed or cleaved in said polymeric matrix, in said analyte, or in said means; and
    c) observing a color transition of said matrix, indicating the presence of the analyte in said sample.

2.  A method according to claim 1, wherein the analyte is an ion and the means allowing said ion to cause a non-chemical change in the polymeric matrix are ionophores.

3.  A method according to claim 2, wherein the ion is a metal cation.

4.  A method according to claim 1, wherein the analyte is a biological ligand and the means allowing said ligand to cause a non-chemical change in the polymeric matrix are provided by a receptor having the capability to bind said ligand, said receptor being linked to a spacer arm capable of non-covalent binding within the lipid domain of said matrix.

**5.** A method according to claim 4, wherein the biological ligand is selected from the group consisting of antibodies, antigens and epitopes and the spacer arm is a peptide or one or more alkyl chains.

**6.** A method according to claim 1, wherein the polymer is polydiacetylene obtained by polymerisation of a monomer selected from the group consisting of tricosadiynoic acid, tricosadiynoic methyl esters, pentacosadiynoic acid and pentacosadiynoic methyl esters.

**7.** A method according to claim 1, wherein the lipids are selected from the group consisting of phospholipids, sphingolipids and ceramides.

**Patentansprüche**

**1.** Methode für das Erfassen des Vorliegens eines Analyten in einer Probe, wobei der Analyt mit Lipiden oder mit einem Polymer nicht chemisch reaktiv ist, das eine Absorptionsbande aufweist, die von einer ersten Wellenlänge im sichtbaren Bereich auf eine zweite Wellenlänge im sichtbaren Bereich verschoben werden kann, welche Methode Folgendes umfasst:

a) das Bereitstellen einer polymeren Matrix umfassend die Lipide und das Polymer in einer wässrigen Lösung;
b) da Einführen der Probe in die polymere Matrix und eine Möglichkeit, die es dem Analyten ermöglicht, eine Änderung hervorzurufen, die von einem Farbübergang in der polymeren Matrix begleitet ist; wobei keine kovalente Bindung in der polymeren Matrix, im Analyten oder in der Möglichkeit gebildet oder gespalten wird; und
c) das Beobachten eines Farbübergangs der Matrix, was das Vorliegen des Analyten in der Probe anzeigt.

**2.** Methode nach Anspruch 1, wobei der Analyt ein Ion ist und die Möglichkeiten, die es dem Ion erlauben, eine nichtchemische Änderung in der polymeren Matrix hervorzurufen Ionophore sind.

**3.** Verfahren nach Anspruch 2, wobei das Ion ein Metallkation ist.

**4.** Methode nach Anspruch 1, wobei der Analyt ein biologischer Ligand ist und die Möglichkeiten, die es dem Liganden erlauben, eine nichtchemische Änderung in der polymeren Matrix hervorzurufen, durch einen Rezeptor bereitgestellt werden, der die Fähigkeit besitzt, den Liganden zu binden, wobei der Rezeptor mit einem Abstandhalterarm verbunden ist, der des nichtkovalenten Bindens innerhalb der Lipiddomäne der Matrix fähig ist.

**5.** Methode nach Anspruch 4, wobei der biologische Ligand aus der Gruppe ausgewählt ist bestehend aus Antikörpern, Antigenen und Epitopen und der Abstandhalterarm ein Peptid oder eine oder mehrere Alkylketten ist.

**6.** Methode nach Anspruch 1, wobei das Polymer Polydiacetylen ist, das durch Polymerisation eines Monomers erhalten wird ausgewählt aus der Gruppe bestehend aus Tricosadiynsäure, Tricosadiynmethylestern, Pentacosadiynsäure und Pentacosadiynmethylestern.

**7.** Methode nach Anspruch 1, wobei die Lipide aus der Gruppe ausgewählt werden bestehend aus Phospholipiden, Sphingolipiden und Ceramiden.

**Revendications**

**1.** Méthode permettant de détecter la présence d'un analyte dans un échantillon, ledit analyte étant chimiquement non réactif avec des lipides ou avec un polymère dont la bande d'absorption peut être déplacée d'une première longueur d'ondes située dans la région visible à une seconde longueur d'onde située dans la région visible, la méthode faisant intervenir :

a) l'utilisation d'une matrice polymère qui comprend lesdits lipides et ledit polymère dans une solution aqueuse ;
b) l'introduction dans ladite matrice polymère dudit échantillon et un moyen permettant au dit analyte de causer

EP 1 161 688 B1

un changement qui est accompagné par une transition de couleur dans ladite matrice polymère, aucune liaison covalente n'étant formée ni rompue dans ladite matrice polymère, ledit analyte ou ledit moyen ; et
c) l'observation d'une transition de couleur de ladite matrice qui est indicatrice de la présence de l'analyte dans ledit échantillon.

2. Méthode selon la revendication 1, où l'analyte est un ion et où le moyen permettant au dit ion de causer un changement non chimique dans la matrice polymère correspond à des ionophores.

3. Méthode selon la revendication 2, où l'ion est un cation métallique.

4. Méthode selon la revendication 1, où l'analyte est un ligand biologique et le moyen permettant au dit ligand de causer un changement non chimique dans la matrice polymère fait intervenir un récepteur qui est capable de lier ledit ligand, ledit récepteur étant lié à un bras espaceur capable d'assurer une liaison non covalente au sein du domaine lipidique de ladite matrice.

5. Méthode selon la revendication 4, où le ligand biologique est sélectionné parmi le groupe consistant en des anticorps, des antigènes et des épitopes et le bras espaceur est un peptide ou une ou plusieurs chaînes alkyles.

6. Méthode selon la revendication 1, où le polymère est un polydiacétylène obtenu par polymérisation d'un monomère sélectionné parmi le groupe consistant en l'acide tricosadiynoïque, les esters de méthyle de l'acide tricosadiynoïque, l'acide pentacosadiynoïque et les esters de méthyle de l'acide pentacosadiynoïque.

7. Méthode selon la revendication 1, où les lipides sont sélectionnés parmi le groupe consistant en des phospholipides, sphingolipides et céramides.

Fig. 1

Fig. 2

Fig. 3

Fig. 4

Fig. 5A

Fig. 5B

Fig. 6

Fig. 7

Fig. 8

Fig. 9

Fig. 10

Fig. 11

A

Fig. 12A

B

Fig. 12B

C

Fig. 12C

175         173         171

chemical shift (ppm)